# EUROPEAN PATENT SPECIFICATION

(11) **EP 1 670 481 B1**
(45) Date of publication and mention of the grant of the patent: **04.08.2010**
(21) Application number: 04766789.4
(22) Date of filing: 15.09.2004
(51) Int. Cl.: A61K 31/58, A61P 1/00

(54) **USE OF CICLESONIDE FOR THE TREATMENT OF INFLAMMATORY BOWEL DISEASES**
VERWENDUNG VON CICLESONID ZUR BEHANDLUNG VON ENTZÜNDLICHEN DARMERKRANKUNGEN
UTILISATION DE CICLESONIDE DANS LE TRAITEMENT DE MALADIES INTESTINALES INFLAMMATOIRES

(30) Priority: 15.09.2003 EP 03020871
(43) Date of publication of application: 21.06.2006
(73) Proprietor: Nycomed GmbH, 78467 Konstanz (DE)
(72) Inventor: ABEL, Florian, 78351 Bodmann-Ludwigshafen (DE); BRUECK-SCHEFFLER, Antje, 78464 Konstanz (DE); DIETZEL, Klaus, 78345 Moos-Weiler (DE); EISTETTER, Klaus, 78465 Konstanz (DE); NAVE, Ruediger, 78464 Konstanz (DE); POSTIUS, Stefan, 78467 Konstanz (DE); VON RICHTER, Oliver, 40822 Mettmann (DE)
(74) Representative: Kratzer, Bernd
(86) International application number: PCT/EP2004/052170
(87) International publication number: WO 2005/025577

(56) References cited:
- WO-A-91/07172
- WO-A-94/22899
- WO-A-99/47144
- US-A- 5 482 934
- MUTLU E A ET AL: "New developments in the treatment of inflammatory bowel disease" EXPERT OPINION ON INVESTIGATIONAL DRUGS 2002 UNITED KINGDOM, vol. 11, no. 3, 2002, pages 365-385, XP009025867 ISSN: 1354-3784
- DENT G: "Ciclesonide Byk Gulden" CURRENT OPINION IN INVESTIGATIONAL DRUGS 2002 UNITED KINGDOM, vol. 3, no. 1, 2002, pages 78-83, XP001179540 ISSN: 1472-4472
- SCHMIDT B M W ET AL: "The new topical steroid ciclesonide is effective in the treatment of allergic rhinitis" JOURNAL OF CLINICAL PHARMACOLOGY, LIPPINCOTT CO, HAGERSTOWN, MD, US, vol. 39, no. 10, October 1999 (1999-10), pages 1062-1069, XP008024248 ISSN: 0091-2700

## Description

### Field of application of the invention

The present invention relates the novel use of ciclesonide in the treatment of inflammatory bowel diseases and to pharmaceutical compositions containing ciclesonide for the treatment of certain inflammatory bowel diseases.

### Known technical background

Inflammatory bowel disease is the term generally applied to two diseases, namely ulcerative colitis and Crohn's disease.

Ulcerative colitis is a chronic inflammatory disease of unknown etiology afflicting only the large bowel and, except when very severe, limited to the bowel mucosa. The course of the disease may be continuous or relapsing, mild or severe. The risk of colon carcinoma has been reported to be elevated after longstanding disease. Ulcerative colitis is curable by total colectomy, which may be needed for acute severe disease or chronic unremitting disease. Most patients with ulcerative colitis are managed medically rather than surgically.

Crohn's disease is also a chronic inflammatory disease of unknown etiology but, unlike ulcerative colitis, it can affect any part of the bowel. Although lesions may start superficially, the inflammatory process extends through the bowel wall to the draining lymph nodes. As with ulcerative colitis, the course of the disease may be continuous or relapsing, mild or severe but, unlike ulcerative colitis it is not curable by resection of the involved segment of bowel. Most patients with Crohn's disease come to surgery at some time, but subsequent relapse is common and continuous medical treatment is usual.

For treatment of acute attacks of ulcerative colitis, glucocorticosteroids such as prednisone or prednisolone acetate are almost invariably used and given parenterally or by mouth for the average acute attack or relapse, or locally, by enema.

US 5643602 is related to oral pharmaceutical compositions comprising budesonide and similar steroids for use in the treatment of inflammatory bowel diseases. Pharmaceutical compositions based on Budesonide for the treatment of inflammatory bowel disease are commercially available (Entocort® and Budenofalck®).

Ciclesonide is a novel inhaled corticosteroid for asthma treatment, which is undergoing clinical evaluation. For ciclesonide low systemic side effects have been observed in clinical studies. Ciclesonide has very low affinity for the glucocorticosteroid receptor but is readily converted to the active metabolite desisobutyryl-ciclesonide by esterases in the lung to provide local activity in the target organ. This activation occurs by ester cleavage at the C21 position of ciclesonide. The affinity of desisobutyryl-ciclesonide to the glucocorticosteroid receptor is approximately 100 times higher than that of ciclesonide. Ciclesonide has a low oral bioavailability in the human system, which is below 1%.

Ciclesonide and various other novel glucocorticoids as well as pharmaceutical formulations for ciclesonide are disclosed in US 5482934.

WO 99/47144 is related to the use of glucocorticoid having a first pass metabolism in the liver of at least 90% for the manufacture of a medicament for treating glomerulonephritis by oral or rectal administration of a pharmacologically effective amount thereof for release in the intestine.

### Description of the invention

Surprisingly it has been found now that ciclesonide is particularly suitable for the treatment of inflammatory bowel diseases. Unexpectedly with regard to above described properties of ciclesonide it has been found that ciclesonide when administered in particular by the oral route has a great potential benefit in the treatment of inflammatory bowel disease.
Due to its high anti-inflammatory activity and low systemic activity ciclesonide provides a significant benefit and advantage in the treatment of inflammatory bowel disease. In addition, ciclesonide exhibits a high rate of systemic clearance, which prevents it from exerting significant systemic effects. This superior therapeutic ratio compared to other steroids makes ciclesonide particularly suitable for the treatment -in particular for a long term treatment- of inflammatory bowel diseases. In particular due to the low systemic effect ciclesonide may be used in maintenance or long term therapy in the treatment of inflammatory bowel disease providing the benefit of less side effects for the patient in need thereof.

In one aspect the present invention therefore relates to the use of ciclesonide in the manufacturing of a medicament (pharmaceutical composition) for the treatment of inflammatory bowel disease. Ciclesonide is the INN for an active compound having the chemical name [11β,16α-(R)]-16,17-[(cyclohexylmethylene)bis(oxy)]-11-hydroxy-21-(2-methyl-1-oxopropoxy)pregna -1,4-diene-3,20-dione. Ciclesonide and its preparation are described in US 5482934. According to the invention, the name "ciclesonide" is understood as meaning not only the pure R epimer of the compound [11β,16α]-16,17-[(cyclohexylmethylene)bis(oxy)]-11-hydroxy-21-(2-methyl-1-oxopropoxy)pregna-1,4-diene-3,20-dione but also R/S epimer mixtures in any desired mixing ratio (that is the compounds [11β,16α(R)]-16,17-[(cyxclohexylmethylene)bis(oxy)]-11-hydroxy-21-(2-methyl-1-oxopropoxy)pregna-1,4diene-3,20-dione and [11β,16α(S)]-16,17-[(cyclohexylmelhylene)bis(oxy)]-11-hydroxy-21-(2-methyl 1-oxopropoxy)pregna-1,4-diene-3,20-dione), those being preferred which essentially consist of R epimers. According to the invention, essentially consisting of R epimers means that the proportion of S epimers in the mixture is less than or equal to 5%, preferably less than or equal to 1%.

Inflammatory bowel disease in connection with the invention refers to chronic non-specific inflammatory conditions of the gastrointestinal tract, of which the two major forms are Crohn's disease and ulcerative colitis. Crohn's disease is characterised by thickened areas of the gastrointestinal wall, with inflammation extending through all layers, deep ulceration and fissuring of the mucosa, and the presence of granulomas; affected areas may occur in any part of the gastrointestinal tract, interspersed with areas of relatively normal tissue; the terminal ileum is frequently involved. Symptoms depend on the site of disease but may include abdominal pain, diarrhoea, fever, weight loss, and rectal bleeding. Extra-intestinal manifestations may include joint inflammation, skin lesions, mouth ulcers, and liver disorders. In ulcerative colitis, disease is confined to the colon and rectum, inflammation is superficial but continuous over the affected area, and granulomas are rare. In mild disease, the rectum alone may be affected (proctitis); in severe disease, ulceration is extensive and much of the mucosa may be lost, with an increased risk of toxic dilatation of the colon, a potentially life-threatening complication. Symptoms include diarrhoea and rectal bleeding. The extra-intestinal manifestations are similar to those of Crohn's disease. Patient in connection with the invention preferably refers to a human suffering from an inflammatory bowel disease as defined above.

In another aspect the invention relates to a medicament (herein also referred to as pharmaceutical composition) comprising a therapeutically effective amount of ciclesonide and a pharmaceutically acceptable carrier and/or excipients according to claim 7. The pharmaceutical composition is suitable for oral administration. Pharmaceutical compositions, which may be mentioned are e.g. tablets, coated tablets, capsules, pellets, granulates, emulsions, suspensions or gels.

Excipients suitable for the desired pharmaceutical composition according to the invention are familiar to the person skilled in the art.

In order for the oral composition containing ciclesonide to be applicable for the treatment of the bowel diseases the composition must be adjusted to this particular purpose.
The transit time through the gastro-intestinal canal for different dosage forms are rather well known. When the dosage form has been emptied from the stomach the transit through the small intestine takes 3 to 5 hours. The residence time in the large intestine is considerably longer, 25 to 50 hours. Ideally, as long as the dosage form remains in the stomach no release should occur. If Crohn's disease in small intestine is going to be treated the release should start after the dosage form has left the stomach and if necessary, should continue during about 3 to 5 hours after the dosage form has left the stomach. If the large intestine is going to be treated the release should ideally start at the distal small intestine oar at caecum, if necessary, followed by slow release of ciclesonide. Ciclesonide must have a chance to reach the inflamed part of the bowel in sufficient concentration and for a sufficient long time to exert it's local action, in the case of Crohn's disease the whole bowel or only the small intestine and in the case of ulcerative colitis the caecum (cecum), colon and the rectum.

In ulcerative colitis, the composition should be formulated so that ciclesonide is released preferentially during the passage of the colon. In Crohn's disease in the ileum the composition should be formulated so that the ciclesonide is released preferentially during the passage of the small intestine.

Such formulations designed for the treatment of inflammatory diseases allowing for targeted release in the small intestine and colon can be prepared by enteric and slow release coating of the units containing ciclesonide. Preferably ciclesonide is contained in a core formulation releasing the drug substance immediately. Such core formulations can be granulates, pellets or tablets. By coating these core formulations with at least one enteric and at least one slow release coating the desired release profile can be obtained. Pharmaceutically acceptable excipients for enteric coating being insoluble at gastric pH but soluble at intestinal pH include cellulose acetate phthalate, hydroxpropylmethyl-cellulose phthalate, polyvinylacetate phthalate and acrylic acid polymers e.g. partly esterified methacrylic acid polymers such as Eudragit L, Eudragit L1 00-55 and Eudragit S. The acrylic polymers can also be used as commercially available aqueous dispersions, e.g. Eudragit FS30D Eudragit L30 D55. These polymers may be used alone or in combination with each other. In order to obtain an acceptable film coat other excipients such as plasticizers, anti-adhesives and antifoaming agents can be added.

The release profile of the coated formulation can be altered by varying the coating thickness as well as the composition of the coating. If slow release of ciclesonide is desired in the targeted regions of the GI tract, mixtures of enteric coatings with slow release coatings can be applied. This can be done by only one single coating layer or in two or more separate layers. The polymers of this coating can be selected from cellulose derivatives and copolymers of methacrylic acid esters such as ethylcellulose and Eudragit NE, Eudragit RL and Eudragit RS. These polymers are also commercially available in form of aqueous dispersions. In order to obtain an acceptable film coat other excipients such as plasticizers, anti-adhesives and antifoaming agents can be added. The release profile of the coated formulation can the altered by varying the coating thickness as well as the composition of the coating.

The core formulation can be prepared employing excipients known for those being skilled in the art: Fillers such as lactose, mannitol, calcium phosphate, microcrystalline cellulose, sucrose, starch, waxes and lipids. Binders such as hydroxypropylmethylcellulose, hydroxypropylcellulose, povidone, starch and its derivatives as well as wetting agents, surfactants and disintegrants. In case of pellets sugar spheres can be used as seed material.

The manufacturing of these formulations is done by processes usually employed for manufacturing of solid oral dosage forms such as granulation by high shear mixing, roller compaction for fluidized bed, preparation of pellets by extrusion spheronisation or layering on seed pellets and compression of powders to tablets. The coating can be done in a fluidized bed process or in a conventional pan or drum coating process by spraying an aqueous or organic fluid.

Suitable dosage forms for targeted release are for example described in US 5643602 and DE 4332394.

The dosage range for treatment of the bowel diseases as hereinbefore defined is suitably within the general dosage range and regimen for glucocorticosteroids in the treatment of bowel disease. Suitable dosage ranges for budesonide are e.g. disclosed in US 5643602. However the person skilled in the art will appreciate that a suitable dosage will depend on factors such as the type of bowel disease, the patient afflicted with the disease, the type of glucocorticosteroid and the route of administration. For example ciclesonide may be administered orally in a daily dosage range of from 1 to 50 mg, preferably from 2 to 25 mg and particularly preferably from 10 to 20 mg. The daily dosage may be administered in a single dosage unit once or the daily dosage may be administered in the form of several dosage units (e.g. twice or three times a day). For example an oral dosage unit may contain 3, 4, 5, 6, 7, 8, 9 or 10 mg of ciclesonide and may be administered once, twice or three times a day.

The production of oral administration forms of ciclesonide for targeted release in the treatment of inflammatory bowel disease is described by way of example below. The following examples illustrate the invention in greater detail, without restricting it.

### Examples

### Example 1

### Step 1: Manufacturing of drug containing pellets

| | |
|---|---|
| Sugar spheres | 10kg |
| Povidone 25 | 0.1kg |
| Polysorbate 80 | 0.01kg |
| Ciclesonide micronized | 0.1kg |

Povidone 25 and polysorbate 80 will be dissolved in water (5 kg). Ciclesonide will be suspended afterwards. This suspension will be sprayed on the sugar spheres employing fluidized bed equipment.

### Step 2: Enteric coating

| | |
|---|---|
| Drug containing pellets from step 1 | 5.50kg |
| Eudragit L | 1.21kg |
| Dibutylphthalat | 0.12kg |
| Talc | 1.00kg |

Eudragit L and dibutylphthalat will be dissolved in isopropanol/water 95:5 15kg. Talc will be suspended in this solution. The coating suspension will be sprayed on the drug containing pellets in a fluidized bed equipment or a coating pan/drum.

### Example 2

### Step 1: Manufacturing of drug containing pellets

Performed as described in example 1, step 1.

### Step 2: Slow release coating

| | |
|---|---|
| Drug containing pellets from step 1 | 5.50kg |
| Eudragit RL | 0.06kg |
| Dibutylphthalat | 0.07kg |
| Talc | 0.10kg |

Eudragit RL will be dissolved in isopropanol/water 9:1 3.66kg. Dibutylphthalat and talc will be added. The coating suspension will be sprayed on the drug containing pellets in a fluidized bed equipment or a coating pan/drum.

### Step 3: Enteric coating

| | |
|---|---|
| Drug containing pellets from step 2 | 5.73kg |
| Eudragit L | 1.21kg |
| Dibutylphthalat | 0.12kg |
| Talc | 1.00kg |

Eudragit L and dibutylphthalat will be dissolved in isopropanol/water 95:5 15kg. Talc will be suspended in this solution. The coating suspension will be sprayed on the drug containing pellets in a fluidized bed equipment or a coating pan/drum.

### Example 3

### Step 1: Manufacturing of drug containing pellets

Performed as described in example 1, step 1.

### Step 2: Enteric and slow release coating

| | |
|---|---|
| Drug containing pellets from step 1 | 5.50kg |
| Polysorbate 80 | 0.01kg |
| Eudragit NE30D | 1.00kg |
| Eudragit S100 | 0.30kg |
| Talc | 0.30kg |

Polysorbate 80 will be dissolved in the Eudragit NE30D dispersion. Eudragit S100 and Talc will be suspended therein. The coating suspension will be sprayed on the drug containing pellets in a fluidized bed equipment or a coating pan/drum.

### Example 4

Tablet and liquid for the preparation of a suspension for rectal application

**Tablet**

| | |
|---|---|
| Lactose monohydrate | 107.4mg |
| Crospovidon | 8.4mg |
| Ciclesonide micronized | 3.0mg |
| Magnesium stearate | 1.2mg |

All ingredients will be blended and subsequently be compressed to tablets.

**Liquid**

| | |
|---|---|
| Sodium chloride | 1.035g |
| Methylparaben | 0.092g |
| Propylparaben | 0.023g |
| Purified water | 115g |

All ingredients will be dissolved in water. The solution will be filled into bottles. Prior to the application a tablet will be added to the liquid. The final suspension for the rectal application will be obtained after disintegration of the tablet.

### Pharmacological Investigations

As an animal model of Inflammatory Bowel Disease, the TNBS-induced Colitis in the rat had been used.

48 h fasted rats (220-250g b.w.) received rectally 0,9 ml of a solution consisting of 30% ethanol, 30 mg/kg TNBS (Trinitrobenzoesulfonic acid) in an oral distance of 8-9 cm from the anus. Compound administration days -1 to +2. Animals were sacrified on day 2.
For intrarectal administration the compounds were solved in 1% ethanol, 10% Propyleneglycol and 20% Cyclodextrin. The used range of concentrations was identical for all compounds. Intrarectal administration schedule: twice daily. Compounds used: Ciclesonide and for comparison, Budesonide and Dexamethasone.

As parameters for the estimation of the pharmacological effects of the compounds, max. colon width had been used. As for the estimation of potential side effects, the weights of thymus and adrenal glands have been registrated.

The efficacy of a treatment was calculated on the reference of controls that received either no TNBS or TNBS rectally, establishing the range of 100 to 0% inhibition, respectively, in the drug screening.

### Results

The results indicate the lowest doses of compounds that impact the indicated parameters to a relevant extent:

| | Compound | max Colon width | Thymus weight | Weight of adrenal glands |
|---|---|---|---|---|
| A | Ciclesonide | 2,3 | n.e. @ 6,9 | 2,3 |
| B | Budesonide | 2,3 - 6,9 | 2,3 | n.e. @ 6,9 |
| C | Dexamethasone | 0,69 | n.t. | n.t. |
| D | - TNBS control | normal | normal | normal |
| E | + TNBS control | increase | decrease | increase |
| F | Compound + TNBS | Inhibition of the effect in E | Amplification of the effect in E | Inhibition of the effect in E |

None of the doses of any of the three compounds tested yielded 100% efficacy in this model. Maximally observed effects on the colon width are between 30 and 60%.

The results demonstrate the efficacy of ciclesonide, attenuating the TNBS-increased colon width starting with 2,3 µmol/animal. At the highest assessed dose of 6,9 µmol/animal, no additional decrease of the TNBS-reduced thymus weight could be detected. Instead, the TNBS-increased adrenal glands weight was attenuated starting with 2,3 µmol/animal ciclesonide.

By contrast, budenoside seems to additionally reduce the thymus weight starting with 2,3 µmol/animal and does not reverse the inhibitory effect of TNBS-treatment on the adrenal glands weight in the assessed pharmacodynamic dose range.

## Claims

1. Use of ciclesonide in the manufacturing of a medicament for the treatment of inflammatory bowel disease.

2. Use according to claim 1, wherein the inflammatory bowel disease is selected from Crohn's disease and ulcerative colitis.

3. Use according to claim 1, wherein the medicament is comprising a therapeutically effective amount of ciclesonide and a pharmaceutically acceptable carrier and/or excipients suitable for oral administration.

4. Use according to claim 1, wherein the pharmaceutical composition allows for targeted release of ciclesonide in the small intestine and/or colon.

5. Use according to claim 1, wherein the pharmaceutical composition is suitable for rectal administration.

6. Use according to claim 1, **characterized in that** ciclesonide is present to more than 95 % in the form of its R epimer in the medicament.

7. Pharmaceutical composition comprising a core formulation containing a therapeutically effective amount of ciclesonide and a pharmaceutically acceptable carrier and/or excipients suitable for oral administration and a combination of one enteric coating and one slow release coating providing a release profile allowing targeted release of ciclesonide in the small intestine and/or colon.

8. Pharmaceutical composition according to claim 7, wherein the core formulation is a granulate, a pellet or a tablet.

9. Pharmaceutical composition according to claim 8, wherein the core formulation is an immediate release formulation.

## Patentansprüche

1. Verwendung von Ciclesonid bei der Herstellung eines Medikaments zur Behandlung von entzündlicher Darmerkrankung.

2. Verwendung gemäß Anspruch 1, wobei die entzündliche Darmerkrankung ausgewählt ist aus Morbus Crohn und ulzerierender Colitis.

3. Verwendung gemäß Anspruch 1, wobei das Medikament eine therapeutisch wirksame Menge Ciclesonid und einen pharmazeutisch verträglichen Träger und/oder Exzipienten, der für die orale Verabreichung geeignet ist, umfasst.

4. Verwendung gemäß Anspruch 1, wobei die pharmazeutische Zusammensetzung die gezielte Freisetzung von Ciclesonid im Dünndarm und/oder Colon erlaubt.

5. Verwendung gemäß Anspruch 1, wobei die pharmazeutische Zusammensetzung für die rektale Verabreichung geeignet ist.

6. Verwendung gemäß Anspruch 1, **dadurch gekennzeichnet, dass** Ciclesonid zu mehr als 95 % in der Form seines R-Epimers in dem Medikament vorhanden ist.

7. Pharmazeutische Zusammensetzung, umfassend eine Kernformulierung, umfassend eine therapeutisch wirksame Menge Ciclesonid und einen pharmazeutisch verträglichen Träger und/oder Exzipienten, der für die orale Verabreichung geeignet ist, und eine Kombination von einem magensaftresistenten Überzug und einem Überzug mit langsamer Freisetzung, die ein Freisetzungsprofil bereitstellt, das die gezielte Freisetzung von Ciclesonid im Dünndarm und/oder Colon erlaubt.

8. Pharmazeutische Zusammensetzung gemäß Anspruch 7, wobei die Kernformulierung ein Granulat, ein Pellet oder eine Tablette ist.

9. Pharmazeutische Zusammensetzung gemäß Anspruch 8, wobei die Kernformulierung eine Formulierung mit sofortiger Freisetzung ist.

## Revendications

1. Utilisation de ciclésonide dans la fabrication d'un médicament pour le traitement d'une affection abdominale inflammatoire.

2. Utilisation selon la revendication 1, dans laquelle l'affection abdominale inflammatoire est choisie parmi la maladie de Crohn et la recto-colite hémorragique.

3. Utilisation selon la revendication 1, dans laquelle le médicament comprend une quantité thérapeutiquement efficace de ciclésonide et un véhicule pharmaceutiquement acceptable et/ou des excipients adaptés pour administration orale.

4. Utilisation selon la revendication 1, dans laquelle la composition pharmaceutique permet la libération ciblée de ciclésonide dans l'intestin grêle et/ou le côlon.

5. Utilisation selon la revendication 1, dans laquelle la composition pharmaceutique est adaptée pour administration rectale.

6. Utilisation selon la revendication 1, **caractérisée en ce que** le ciclésonide est présent à plus de 95 % sous la forme de son épimère R dans le médicament.

7. Composition pharmaceutique comprenant une formulation de noyau contenant une quantité thérapeutiquement efficace de ciclésonide et un véhicule pharmaceutiquement acceptable et/ou des excipients adaptés pour administration orale et une combinaison d'un enrobage entérique et un enrobage à libération lente produisant un profil de libération permettant la libération ciblée de ciclésonide dans l'intestin grêle et/ou le côlon.

8. Composition pharmaceutique selon la revendication 7, dans laquelle la formulation de noyau est un granule, une pastille ou un comprimé.

9. Composition pharmaceutique selon la revendication 8, dans laquelle la formulation de noyau est une formulation à libération immédiate.
